# EUROPEAN PATENT SPECIFICATION

(11) **EP 1 283 261 B1**
(45) Date of publication and mention of the grant of the patent: **06.08.2008**
(21) Application number: 01925961.3
(22) Date of filing: 26.04.2001
(51) Int. Cl.: C12N 15/09, C12Q 1/02, C12N 1/19

(54) **METHOD OF SCREENING DRUG ACTING ON CELL WALL**
VERFAHREN ZUM SCREENING VON AUF ZELLWÄNDE WIRKENDEN ARZNEISTOFFEN
PROCEDE DE CRIBLAGE DE MEDICAMENTS AGISSANT SUR LA PAROI CELLULAIRE

(30) Priority: 01.05.2000 JP 2000132041
(43) Date of publication of application: 12.02.2003
(73) Proprietor: Daiichi Sankyo Company, Limited, Chuo-ku Tokyo 103-8426 (JP)
(72) Inventor: KITAMURA, Akihiro, Daiichi Pharmaceutical Co.,Ltd, Tokyo 134-8630 (JP); SOMEYA, Kazuhiko, Daiichi Pharmaceutical Co., Ltd, Tokyo 134-8630 (JP); NAKAJIMA, Ryohei, Daiichi Pharmaceutical Co., Ltd, Tokyo 134-8630 (JP)
(74) Representative: Polypatent
(86) International application number: PCT/JP2001/003630
(87) International publication number: WO 2001/083733

(56) References cited:
- WO-A-99/67419
- LU CHA-FEN ET AL: "Glycosyl phosphatidylinositol-dependent cross-linking of alpha-agglutinin and beta-1,6-glucan in the Saccharomyces cerevisiae cell wall" 1995, JOURNAL OF CELL BIOLOGY, VOL. 128, NR. 3, PAGE(S) 333-340 , XP002336721 ISSN: 0021-9525 * page 339, column 1, paragraph 2 * * abstract *
- LU CHA-FEN ET AL: "A pathway for cell wall anchorage of Saccharomyces cerevisiae alpha-agglutinin" MOLECULAR AND CELLULAR BIOLOGY, vol. 14, no. 7, 1994, pages 4825-4833, XP002390482 ISSN: 0270-7306
- KOLLAR ROMAN ET AL: "Architecture of the yeast cell wall: beta(1 fwdarw 6)-glucan interconnects mannoprotein, beta-1( fwdarw 3)-glucan, and chitin" JOURNAL OF BIOLOGICAL CHEMISTRY, vol. 272, no. 28, 1997, pages 17762-17775, XP002390483 ISSN: 0021-9258
- SHAHINLAN S ET AL: "BETA-1,6-GLUCAN SYNTHESIS IN SACCHAROMYCES CEREVISIAE" MOLECULAR MICROBIOLOGY, BLACKWELL SCIENTIFIC, OXFORD, GB, vol. 35, no. 3, 2000, pages 477-489, XP001053225 ISSN: 0950-382X
- POPOLO L ET AL: "The Gas1 glycoprotein, a putative wall polymer cross-linker" BBA - GENERAL SUBJECTS, ELSEVIER SCIENCE PUBLISHERS, NL, vol. 1426, no. 2, 6 January 1999 (1999-01-06), pages 385-400, XP004276265 ISSN: 0304-4165
- LEIDICH S D ET AL: "TEMPERATURE-SENSITIVE YEAST GPI ANCHORING MUTANTS GPI2 AND GPI3 AREDEFECTIVE IN THE SYNTHESIS OF N-ACETYLGLUCOSAMINYL PHOSPHATIDYLINOSITOL" JOURNAL OF BIOLOGICAL CHEMISTRY, AMERICAN SOCIETY OF BIOLOCHEMICAL BIOLOGISTS, BIRMINGHAM,, US, vol. 270, no. 22, 2 June 1995 (1995-06-02), pages 13029-13035, XP002014328 ISSN: 0021-9258
- KENJI HAMADA ET AL.: 'Amino acid sequence requirement for efficient incorporation of glycosylphosphatidylinositol-associated proteins into the cell wall of saccharmyces cerevisiae' THE JOURNAL OF BIOLOGICAL CHEMISTRY vol. 273, no. 41, October 1998, pages 26946 - 26953, XP002944785
- L. HELEEN ET AL.: 'In silico identification of glycosyl-phosphatidylinositol-anchored plasma-membrane and cell wall proteins of saccharomyces cerevisiae' YEAST vol. 13, 1997, pages 1477 - 1489, XP002944786
- BRENDAN P. CORMACK ET AL.: 'FACS-optimized mutants of the green fluorescent protein (GFP)' GENE vol. 173, 1996, pages 33 - 38, XP002944787
- MAARTEN P. SCHREUDER ET AL.: 'Targeting of a heterologous protein to the cell wall of saccharomyces cerevisiae' YEAST vol. 9, 1993, pages 399 - 409, XP002944788

## Description

### Technical Field

The present invention relates to a microorganism having a reporter protein such as a fluorescent protein fixed on a cell wall or cell membrane as a GPI-anchored protein and a method for screening an agent acting on cell wall by utilizing the aforementioned microorganism.

### Background Art

Increasing tendency of incidence of deep fungal infections has been seen as the number of compromised patients increases, and therefore, effective therapeutic agents have been desired. Currently, only five antifungal agents for deep fungal infections have been launched in the market in Japan. Among them, three agents are azole-type agents (miconazole, fluconazole and itraconazole). Fluconazole, a most typical agent, has only a fungistatic action. Moreover, with increase of amount of the agent used, appearance of resistant fungi is concerned. Amphotericin B, a polyene antibiotic having a potent fungicidal effect, is highly toxic, and the agent cannot be always used safely. From these reasons, antifungal treatment of patients with deep fungal infections often results in a poor satisfactory level, and thus demands for novel fungicidal and fungiselective agents are urgent.

A cell wall which characteristically exists in fungal cells is an attractive target from a viewpoint of selectivity. In yeast, for example, major saccharide polymers constituting the cell wall include (1,3)- *β* -glucan, (1,6)- *β* -glucan, chitin and mannan. Among synthetic pathways of these saccharide polymers, the synthetic pathway of mannan commonly exists in animal cells and fungal cells and each biosynthetic pathway has high commonness, and therefore, it is considered generally difficult, although not absolutely impossible, to find a target specific to fungi. In the synthetic pathways of (1,3)- *β* -glucan and that of chitin, existence of enzymes specific to fungi and essential for their growth, such as the FKS gene group and the CHS gene group, has been elucidated, and research and development of antifungal agents targeting the enzymes are being conducted. The (1,6)- *β* -glucan synthetic pathway is considered as specific to fungi, and existence of enzymes believed to be essential for growth of fungi has been elucidated based on results of genetic analyses. However, no assay system at an enzyme level has been established, and accordingly, no inhibitor against these enzymes has been reported. For this reason, no antifungal agent inhibiting this synthetic pathway has been known to date.

In general, proteins collectively called as GPI-anchored proteins extensively exist in eukaryotic cells. These are fixed on cell membranes via GPI anchors (Ferguson, M.A., et al., Ann. Rev. Biochem., 57, pp.285-320, 1988). Each GPI-anchored protein has relatively hydrophobic signal peptide regions at both ends of the N- and C-terminals. These signals are cleaved by post-translational modification, and with addition of a GPI anchor to the C-terminus, the protein is fixed on the ER (rough endoplasmic reticulum) membrane. Then, the GPI-anchored protein fixed on the ER membrane is transported on the membrane, and then further fixed on the cell membrane (Ferguson, M.A., et al., Ann. Rev. Biochem., 57, pp.285-320, 1988; Lu, C.F., et al., Mol. Cell Biol., 14, pp.4825-4833, 1994).

In *Saccharomyces cerevisiae*, a part of the GPI anchor is further cleaved from some of the GPI-anchored proteins fixed on the cell membrane, and then the protein is further fixed on the cell wall via (1,6)- *β* -glucan as an anchor (Lu, C.F., et al., Mol. Cell Biol., 14, pp.4825-4833, 1994; Kollar, R., et al., J. Biol. Chem., 272, pp.17762-17775, 1997).

This means that, in *Saccharomyces cerevisiae*, two kinds of GPI-anchored proteins exist; one is fixed on the cell membrane and the other in the cell wall. This difference in localization is expected to be regulated by the difference in the signal peptide at the C-terminus (Hamada, K., et al., Mol. Gen. Genet., 258, pp.53-59, 1998; Caro, L., Yeast, 13, pp.1477-1489, 1997). Recently, creation of an yeast having an arbitrary exogenous protein fixed on the cell wall (arming yeast) was reported by utilizing the localization mechanism of proteins on cell walls (Varrt, J.M.V.D., et al., Appl. Environ. Microbiol., 63, pp.615-620, 1997; Murai, T., et al., Appl. Environ. Microbiol., 63, pp.1362-1366, 1997).

As described above, (1,6)- *β* -glucan has a function as an anchor for fixing the GPI-anchored proteins on the cell walls, and analytical results so far obtained reveal that, when biosynthesis of (1,6)- *β* -glucan is inhibited by gene disruption, these proteins are extracellularly released (Lu, C.F., et al., Mol. Cell Biol., 14, pp.4825-4833, 1994; Lu, C.F., et al., J. Cell. Biol., 128, pp.333-340, 1995).

Recently, Tsuchiya et al. reported construction of an expression system of a reporter protein bound with staphylococcus cell wall peptide glycan (The Pharmaceutical Society of Japan, The 120th Annual Meeting, Abstracts 2, p.153, Lecture No. 30 [PB] 15-71). This system comprises cephalosporinase as a reporter protein anchored on a cell wall of gram-positive bacterium. However, application of this system has not been clarified, and the publication neither suggests nor teaches that the system can be used for screening of an agent acting on cell wall.

### Disclosure of the Invention

An object of the present description is to provide a method for screening an agent acting on cell wall, preferably a method for efficiently screening an agents having a selective inhibitory action on a cell wall, for example, a selective inhibitory action on a biosynthetic enzyme of (1,6)-β-glucan that constitutes a cell wall, by using microorganisms. Further, another object of the present invention is to provide a microorganism used for the aforementioned screening method.

The inventors of the present description conducted various research to achieve the aforementioned objects. As a result, they found that an agent having a selective inhibitory action on a cell wall, for example, an agent having a selective inhibitory action on an enzyme for biosynthesis of (1,6)- *β* -glucan that constitutes the cell wall, should be successfully screened by preparing two kinds of yeasts each having an easily detectable protein (reporter protein) fixed on the cell membrane or cell wall (referred to as "membrane-type arming yeast" and "wall-type arming yeast", respectively), and using a criterion that a release of the reporter protein is occurred substantially only from the wall-type arming yeast. The present invention was achieved on the basis of these findings.

The present description thus provides a method for screening an agent acting on cell wall, which comprises the steps of:
(1) culturing each of a microorganism having a reporter protein fixed on a cell wall as a GPI-anchored protein and a microorganism having a reporter protein fixed on a cell membrane as a GPI-anchored protein in the presence of a test agent;
(2) determining the reporter protein released into each culture fluid of the microorganism cultured; and
(3) judging that the test agent is an agent having a selective inhibitory action on cell wall when the reporter protein is released from the microorganism having the reporter protein fixed on the cell wall into the culture fluid and the reporter protein is not substantially released from the microorganism having the reporter protein fixed on the cell membrane into the culture fluid.

In the aforementioned microorganisms, the reporter protein is fixed on the cell wall or cell membrane via a GPI anchor. The aforementioned microorganisms are thus microorganisms having a GPI-anchored protein fixed on the cell wall or cell membrane in which a protein as a GPI-anchored protein is used as a reporter protein. In other words, the aforementioned microorganism has a reporter protein fixed on the cell wall or cell membrane via the GPI anchor. In the specification, the term "GPI-anchored protein" is not used to mean any specific protein and should not be construed in any limitative sense.

As preferred embodiments of the aforementioned description provided are the aforementioned method, which uses a microorganism having a reporter protein fixed to (1,6)- *β*-glucan of the cell wall; the aforementioned method, wherein the microorganism is yeast; the aforementioned method, wherein the reporter protein is a fluorescent protein; the aforementioned method, wherein the fluorescent protein is a green fluorescent protein; the aforementioned method, wherein the inhibitory action on the cell wall is an inhibitory action against a biosynthetic process and/or a biosynthetic enzyme of the cell wall; and the aforementioned method, wherein the agent acting on the cell wall is an antifungal agent.

From another aspect, the present description provides a microorganism having a reporter protein fixed on a cell wall as a GPI-anchored protein. According to a preferred embodiment of the aforementioned microorganism, the reporter protein is fixed to (1,6)-β-glucan of the cell wall. The present description also provides a microorganism having a reporter protein fixed on a cell membrane as a GPI-anchored protein. These microorganisms are preferably yeasts and can be used for a screening of an agent acting on a cell wall, preferably an antifungal agent. According to a more preferred microorganism, the reporter protein is a fluorescent protein, and the fluorescent protein is a green fluorescent protein. As particularly preferred microorganisms, provided are a yeast AY-14 strain having a green fluorescent protein fixed on the cell wall as a GPI-anchored protein and a yeast AY-15 strain having a green fluorescent protein fixed on the cell membrane as a GPI-anchored protein. The present description further provides use of the microorganisms for a screening of an agent acting on a cell wall.

The present description further provides a set of microorganisms used for a screening of an agent acting on a cell wall, which comprises:
(1) a microorganism having a reporter protein fixed on a cell wall as a GPI-anchored protein; and
(2) a microorganism having a reporter protein fixed on a cell membrane as a GPI-anchored protein.

According to a preferred embodiment of the aforementioned description, provided is a set including microorganisms having a reporter protein fixed to (1,6)-β-glucan of the cell wall. The agent acting on a cell wall is preferably an antifungal agent, and the microorganisms are preferably yeasts. Further, the reporter protein is preferably a fluorescent protein, more preferably a green fluorescent protein. As a particularly preferred set, a set of the AY-14 strain and the AY-15 strain is provided.

Further, the present description provides a gene used for fixing a reporter protein as a GPI-anchored protein on a cell wall or cell membrane of a microorganism, preferably an yeast. Preferred genes (oligonucleotides or polynucleotides) are specifically described in the example of the present specification, and examples include, for example, the gene specified by the nucleic acid sequence of SEQ ID NO: 1 or 2 in Sequence Listing or a gene which has a nucleic acid sequence corresponding to the aforementioned nucleic acid sequence including substitution, insertion and/or deletion of several nucleic acid residues and has substantially the same function as that of the gene specified by the nucleic acid sequence of SEQ ID NO: 1 or 2 in Sequence Listing. The present description further provides a recombinant vector containing any of these genes and a microorganism, preferably an yeast, transformed with the recombinant vector.

### Brief Description of the Drawings

Fig. 1 shows the structures of major plasmids (pEAC and pUAC) used in the example.
Fig. 2 shows results of time course measurement of GFP release based on fluorescence intensity in the culture fluid and the culture supernatants of the AY-5 strain and the AY-16 strain cultured in test tubes.

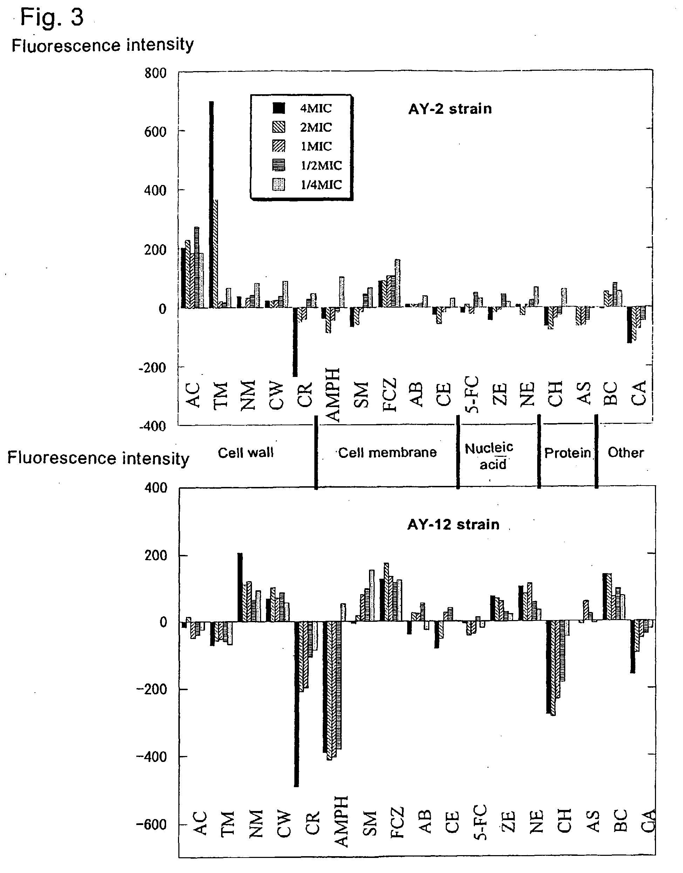

Fig. 3 shows GFP releasing effects by available antifungal reagents. Each macromolecule mainly inhibited is parenthesized. The upper graph shows the results obtained by use of the AY-2 strain, and the lower graph shows the results obtained by use of the AY-12 strain. Agents shown from the left are aculeacin A (AC), tunicamycin (TM), nikkomycin Z (NM), calcofluor white (CW), Congo red (CR), amphotericin B (AMPH), salinomycin (SM), fluconazole (FCZ), aureobasidin (AB), cerulenin (CE), flucytosine (5-FC), zeocin (ZE), netropsin (NE), cycloheximide (CH), azaserine (AS), bromoconduritol (BC) and caffeine (CA).

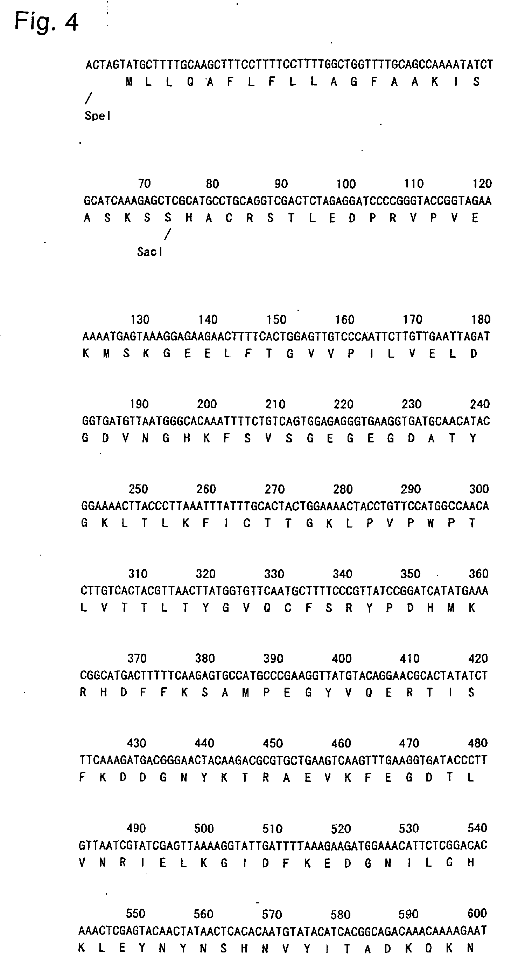

Fig. 4 shows the nucleotide sequence (number of nucleotides: 1116) of the gene introduced into the AY-15 strain, recognition sites of the restriction enzymes and amino acid sequence (one letter code) encoded by the gene. The figure shows the nucleotides up to the nucleic acid number 600.

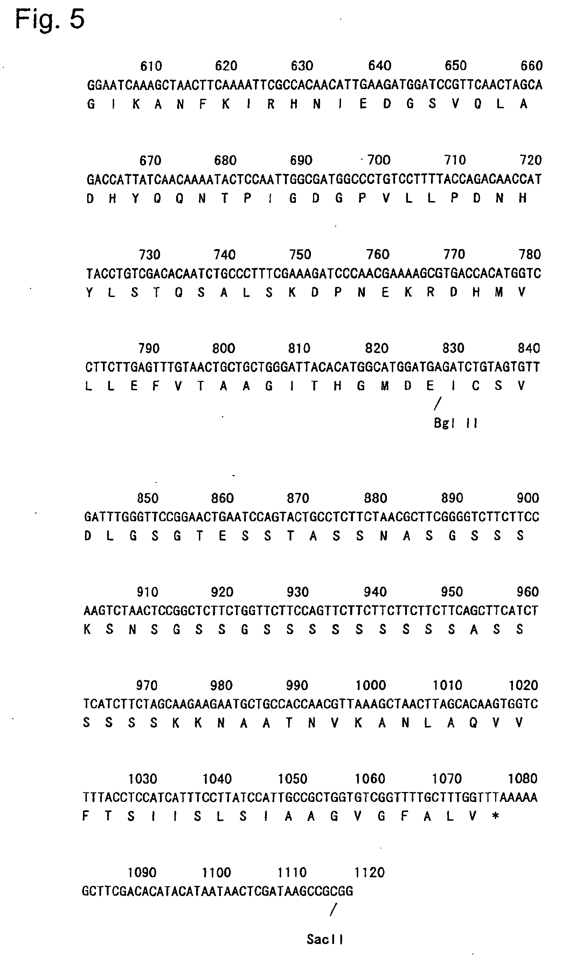

Fig. 5 shows the nucleotide sequence (number of nucleotides: 1116) of the gene introduced into the AY-15 strain, recognition sites of the restriction enzymes and amino acid sequence (one letter code) encoded by the gene. The figure shows the nucleotides of the nucleic acid numbers from 601 to 1116.

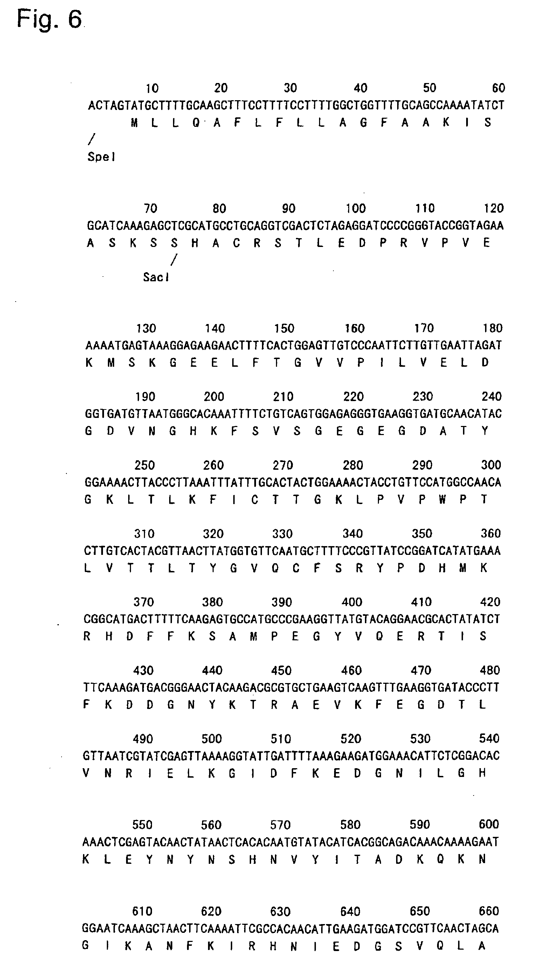

Fig. 6 shows the nucleotide sequence (number of nucleotides: 1236) of the gene introduced into the AY-14 strain, recognition sites of the restriction enzymes and amino acid sequence (one letter code) encoded by the gene. The figure shows the nucleotides up to the nucleic acid number 660.

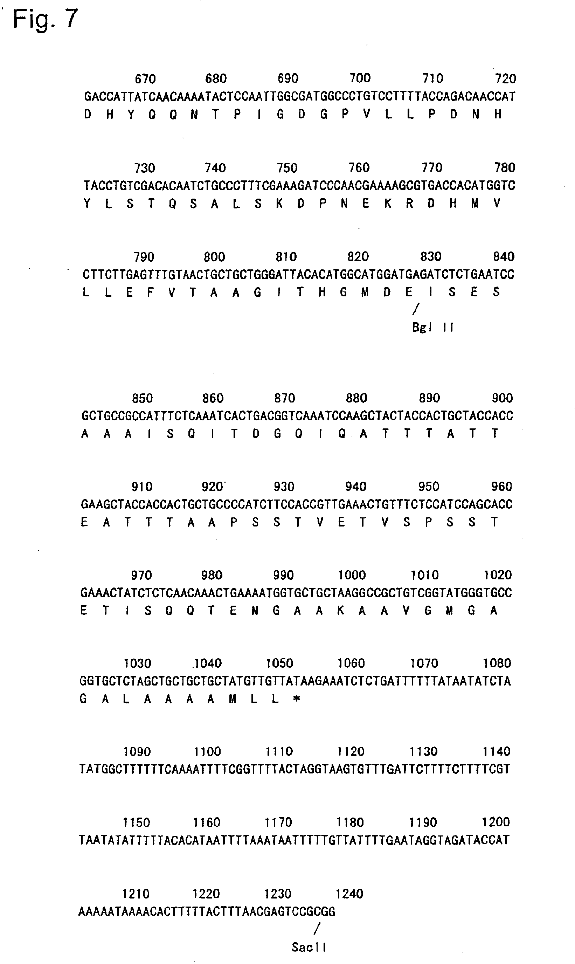

Fig. 7 shows the nucleotide sequence (number of nucleotides: 1236) of the gene introduced into the AY-14 strain, recognition sites of the restriction enzymes and amino acid sequence (one letter code) encoded by the gene. The figure shows nucleotides of the nucleic acid numbers from 661 to 1236.

### Best Mode for Carrying out the claimed method.

Further described is a method is for a screening of an agent acting on cell wall, which is characterized to comprise the steps of:
(1) culturing each of a microorganism having a reporter protein fixed on a cell wall as a GPI-anchored protein and a microorganism having a reporter protein fixed on a cell membrane as a GPI-anchored protein in the presence of a test agent;
(2) determining the reporter protein released into each culture fluid of the microorganism cultured; and
(3) judging that the test agent is an agent having a selective inhibitory action on the cell wall when the reporter protein is released from the microorganism having the reporter protein fixed on the cell wall into the culture fluid and the reporter protein is not substantially released from the microorganism having the reporter protein fixed on the cell membrane into the culture fluid.

Type of the agent acting on cell wall which is a subject of screening of the method of the present invention is not particularly limited. The method of the present description can be utilized for a screening of an agent having a selective inhibitory action on cell wall, preferably a selective inhibitory action on biosynthesis (i.e., an agent having an inhibitory action on the biosynthesis of cell wall, but not substantially having an inhibiting action or a nonspecific inhibitory action on the biosynthesis of cell membrane (e.g., that of detergent)). For example, the method can be preferably used for a screening of an agent having an inhibitory action on the biosynthesis of (1,3)-β-glucan and/or (1,6)-β-glucan, which are major saccharide polymers constituting cell walls, or an agent having an inhibitory action against one or more enzymes involved in biosyntheses of these polymers.

In the present specification, the wording "acting on a cell wall" means, for example, to act on one or more of the following enzymes and interfere with actions or functions of the enzymes.
(a) enzymes for synthesis of components constituting cell wall (saccharide polymers) of microorganisms;
(b) enzymes assisting actions of enzymes for synthesis of components constituting cell walls (saccharide polymers) of microorganisms;
(c) enzymes inhibiting the functions of enzymes for synthesis of components constituting cell walls (saccharide polymers)of microorganisms;
(d) processes required for the enzymes belonging to the class of the aforementioned (b) and (c) to assist or inhibit the actions of the enzyme for synthesis of the aforementioned (a), and enzymes involved in the processes;
(e) processes acting in crosslinking between different saccharide polymers and enzymes involved in the processes;
(f) processes for anchoring components constituting cell walls on the cell walls (e.g., proteins present in the GPI cell walls) besides the saccharide polymers, and enzymes involved in the processes;
(g) processes of normal construction of saccharide polymers in cell walls and enzymes involved in the processes;
(h) processes and pathways for regulating synthesis of cell walls and enzymes involved in the processes;
(i) processes acting in cell division of microbial cells and enzymes involved in the processes;
(j) processes of changing the shapes of microbial cell walls and enzymes involved in the processes;
(k) processes of digesting constructed cell walls of microbial cells and enzymes involved in the processes;
(l) processes in which microorganisms significantly change cell wall compositions in response to changes in the external environment and enzymes involved in the processes; and
(m) other processes of construction and synthesis of microbial cell walls and change of cell wall structures, besides the aforementioned examples, and enzymes involved in the processes.

Further, the wording "having a selective inhibitory action on cell wall" means to have a selective inhibitory action on the events described in the aforementioned (a) to (m) including synthesis, decomposition and the like of cell walls. In the specification, the term "agent" is used so as to encompass any substances having a biological action including low molecular compounds to macromolecular compounds as well as natural substances, proteins or a part thereof, nucleic acids such as oligonucleotides or polypeptides and the like. The term "agent" should not be construed in any limitative sense, and the term should be construed in its broadest sense.

The method as claimed can be most preferably used for a screening of an agent having an inhibitory action on the biosynthesis of (1,6)-β-glucan, or an agent having an inhibitory action on one or more enzymes involved in the biosynthesis of (1,6)-β-glucan. Specific examples of the agent acting on a cell wall include antifungal agents and the like. However, agents that can be screened by the method of the present invention are not limited to antifungal agents.

The reporter proteins fixed on a cell wall or cell membrane of microorganism are not particularly limited so long as they are proteins that can be detected by ordinary means, for example, spectroscopic means such as fluorometry or biochemical means such as enzymatic reactions. For example, a fluorescent protein and the like can be preferably used. For example, a green fluorescent protein (GFP) or a mutant thereof (EGFP: Dormack, B.P., et al., Gene, 173, pp.33-38, etc.) is preferably used. In the specification, the term "green fluorescent protein" is used so as to encompass GFP and mutants thereof. The types of the microorganisms are not particularly limited so long as they are eukaryotic cell microorganisms having a GPI-anchored protein. For example, yeasts such as *S*. *cerevisiae* is preferably used.

In yeast, it is known that a part of some of GPI-anchored proteins fixed on cell membrane are further fixed on the cell wall via (1,6)-β-glucan as an anchor (Lu, C.F., et al., Mol. Cell Biol., 14, pp.4825-4833, 1994; Kollar, R., et al., J. Biol. Chem., 272, pp.17762-17775, 1997). In addition, a method is known for fixing an enzyme on a cell wall of microorganism by producing a fusion protein (Chris et. al, International Patent Unexamined Publication in Japanese (KOHYO) No. 7-508652). On the basis of these findings, methods for fixing an exogenous protein on a cell wall of an yeast have been developed (Varrt, J.M.V.D., et al., Appl. Environ. Microbiol., 63, pp.615-620, 1997; Murai, T., et al., Appl. Environ. Microbiol., 63, pp.1362-1366, 1997). The GPI-anchored protein is also referred to as GPI anchor-type protein, and may also sometimes be referred to as glycosylphosphatidylinositol anchor type protein, phosphatidylinositol anchor type protein, PI anchor type protein or the like.

When yeasts are used as the microorganisms, an yeast in which a reporter protein is fixed on a cell wall or cell membrane as the GPI-anchored protein can be prepared according to the methods described in the aforementioned publications. Specific procedures thereof are described in the example of the present specification, and accordingly, those skilled in the art can produce desired microorganisms according to the methods described in the aforementioned publications and the procedures specifically disclosed in the specification.

In the claimed method, a microorganism having a reporter protein fixed on a cell wall as a GPI-anchored protein and a microorganism having a reporter protein fixed on a cell membrane as a GPI-anchored protein are each cultured in the presence of a test agent, and the reporter proteins released into the culture fluid are determined. The conditions including type of the medium, a temperature, a cultivation period of time and the like can be appropriately chosen depending on the types of the microorganisms used. A specific culture method is specifically described in the example of the specification where an yeast is used as the microorganism. For the measurement of the reporter protein, an appropriate method can be chosen depending on the type and properties of the reporter protein. For example, when a green fluorescent protein is used as the reporter protein, release of the reporter protein can be determined by measuring the fluorescent spectrum in the culture fluid.

In the aforementioned culture step, when release of the reporter protein into the culture fluid is observed, it can be interpreted that the cell wall or cell membrane of the microorganism was damaged by an action of the test agent, and it is generally considered that the cell wall or cell membrane is damaged due to inhibition of biosynthesis of the cell wall or cell membrane, which results in the release of the reporter protein (Lu, C.F., et al., Mol. Cell Biol., 14, pp.4825-4833, 1994; Lu, C.F., et al., J. Cell. Biol., 128, pp.333-340, 1995). For example, when an agent that selectively damages a cell wall is screened as an agent having selective toxicity for fungi, a criterion that the reporter protein is released from the microorganism having the reporter protein fixed on the cell wall into the culture fluid, whilst the reporter protein is not substantially released from the microorganism having the reporter protein fixed on the cell membrane into the culture fluid, and an agent that satisfies the criterion can be chosen. An agent determined as positive for the criterion can be estimated as an agent having a selective inhibitory action on the biosynthesis of the cell wall.

The present invention will be explained more specifically with reference to the following example. However, the scope of the present invention is not limited to this example.

### Example

### (1) Materials and methods

### (A) Strains used and transformation

*Escherichia coli* strains JM109, TOP10F' and DH5α, and *S. cerevisiae* strains YPH500 (MATα ade2, his3, leu2, lys2, trp1, ura3), IFO 0565, IFO 1226 and KRE6-disrupted strain were used. Transformation was performed according to a known method or by using a yeast transformation kit (Invitrogen, Carlsbad, CA, USA) according to the packaged manual.

### (B) Agents used

The agents shown below were used.

**Table 1**

| Agent | Acronym | Action |
|---|---|---|
| Aculeacin A | AC | Inhibition of cell wall ((1,3)-β-glucan) synthesis |
| Tunicamycin | TM | Inhibition of cell wall (N-type saccharide chain) synthesis |
| Nikkomycin Z | NM | Inhibition of cell wall (chitin) synthesis |
| Calcofluor white | CW | Physical adsorption to cell wall |
| Congo red | CR | Physical adsorption to cell wall |
| Amphotericin B | AMPH | Damage of cell membrane (binding to ergosterol) |
| Fluconazole | FCZ | Inhibition of cell membrane (ergosterol) synthesis |
| Aureobasidin | AB | Inhibition of cell membrane (sphingolipid) synthesis |
| Cerulenin | CE | Inhibition of cell membrane (fatty acid) synthesis |
| Salinomycin | SM | Damage of cell membrane (ionophore) |
| Flucytosine | 5-FC | Inhibition of nucleic acid synthesis |
| Zeocin | ZE | Binding to nucleic acid |
| Netropsin | NE | Inhibition of nucleic acid synthesis |
| Cycloheximide | CH | Inhibition of protein synthesis |
| Azaserine | AS | Inhibition of protein synthesis |
| Bromoconduritol | BC | Inhibition of mannosidase |
| Caffeine | CA | Intracellular information transmission (c-AMP) inhibition |

### (C) Oligonucleotides

The following oligonucleotides were used in the experiment. M13 universal primer and M13 reverse primer were purchased from Pharmacia, and the other oligonucleotides were synthesized for use. These oligonucleotides are encompassed within the scope of the genes of the present invention used to fix a reporter protein on a cell wall or cell membrane of microorganism as a GPI-anchored protein.
GFP-SM2 (5'-GGCATGGATGAGATCTACAAATAATG-3')
GFP-SM3 (5'-CATGATTACGCCGAGCTCGCATGCCTG-3')
GFP-SM4 (5'-CAACACTTGTCACTACGTTAACTTATGGTGTTCAATG-3')
YEX-SM2 (5'-CCTGTGATTTCTCCAGCTCGAATTC-3')
YEX-SM3 (5'-GATTCATTAATGCATGCTGGCACGACAGG-3')
YEX-SM4 (5'-GATTCATTAATGCAGCTGGCACGAC-3')
YEX-SM5 (5'-CTCACGGTATCGCCCTCGAGATCTCTGAATCC-3')
YEX-SM6 (5'-GAGACCCTCTTCTGAGCTCTCTGAATCC-3')
YEX-SM7 (5'-AAACCAAAAGATCGACTAGTATAAAATGAATATA-3')
YEX-SM8 (5'-CATTAATGCATGCTGGCACGAC-3')
YEX-SM9 (5'-CTTTAACGAGTCCGCGGATTTCTCCAGCTCG-3')
SUC2-sen1 (5'-GCACTAGTATGCTTTTGCAAGCTTTCCTTTTC-3')
SUC2-anti2 (5'-GCGAGCTCTTTGATGCAGATATTTTGGCTGCAA-3')
GAS1-sen1 (5'-GCAGATCTGTAGTGTTGATTTGGGTTCCGG-3')
GAS1-anti3 (5'-GCCCGCGGCTTATCGAGTTATTATGTATGTGTCGAAGC-3')
CWP2-sen1 (5'-GCAGATCTACTTTGTTGCCGCTGAATCCG-3')
CWP2-anti1 (5'-GCGAATTCGAGAAATCACAGGACTCGTTAAAG-3')
MEL1-sen1 (5'-GCGAATTCGAGAGCAACGGTAATAAAAGCAACGACG-3')
MEL1-sen3 (5'-CGGAGCTCGGTGTCTCCGAGTTACAATGGC-3')
MEL1-anti1 (5'-GCAGATCTAGAAGGCGATACCGTGAGCTGGAAC-3')
MEL1-anti2 (5'-CGGAGCTCCATCAATACTTCTCGCCCTGCT-3')
MEL1-anti3 (5'-GCAGATCTAAGAAGAGGGTCTCAACCTATAGAAG-3')
M13 universal primer and M13 reverse primer

### (D) Plasmids

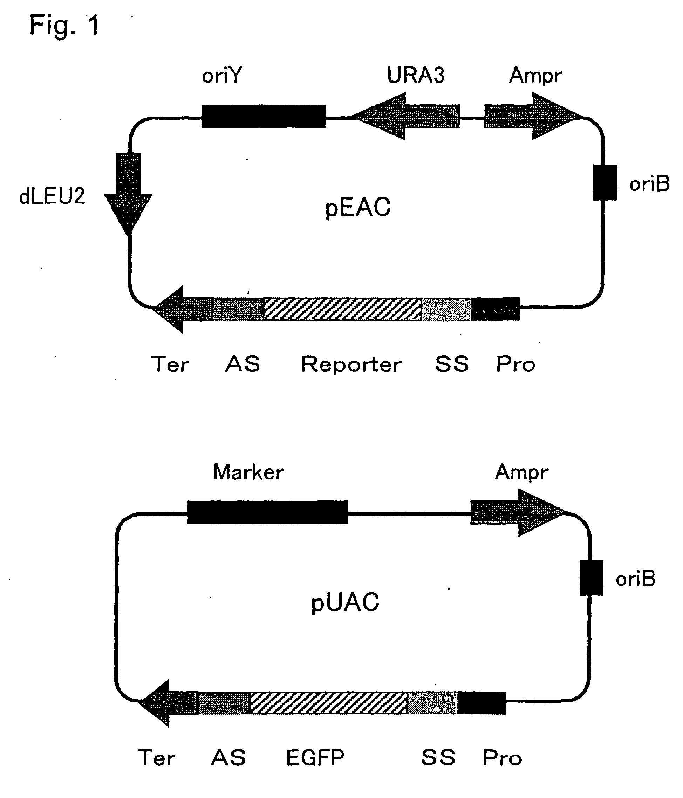

By using pUC19, YEp13, YEp24 (for these, see Pouwels, P.H. et al., Cloning vectors, Elsevier Science Publishers B.V., 1985), pYPR2831 (Horiuchi, H. et al., Agric. Biol. Chem., 54, 1771-1779, 1990), pGFPuv (Clontech, Palo Alto, CA, USA) and pYEX-S1 (Amrad, Victoria, Australia), the following plasmids were prepared and used. These plasmids include recombinant vectors containing the genes of the present invention used to fix a reporter protein on a cell wall or cell membrane of microorganism as a GPI-anchored protein. pGEM-T Vector System (Promega, Madison, WI, USA) was used for subcloning of PCR products, and Transformer Site-Directed Mutagenesis Kit (Clontech) was used for introduction of mutations. Recovery of DNA fragments from agarose, dephosphrylation, blunt-ending, ligation and digestion with restriction enzymes were performed according to conventional methods. The structures of major plasmids are shown in Fig. 1. In the figure, oriB represents the replication origin of *Escherichia coli*, oriY represents the replication origin of baker's yeast, Ampr represents ampicillin resistance gene, dLEU2 represents a partially deficient marker of LEU2, Pro represents a phosphoglycerate kinase promoter, SS represents a secretory signal, and Ter represents a phosphoglycerate kinase terminator.
pUXS1: A fragment obtained by digesting YEp24 with HindIII was inserted into the HindIII site of pUC19.
pUXS2: A fragment obtained by digesting YEp13 with XhoI and Sail was inserted into the SalI site of pUC19.
pUAC1: A part of CWP2 (Varrt, J.M. et al., J. Bacteriol., 177, 3104-3110, 1995) was amplified by PCR (template: YPH500 strain chromosomal DNA, primers: CWP2-senl and CWP2-anti1) and subcloned into pGEM-T.
pUACla: pUACl was digested with EcoRI and PstI and then self-ligated.
pUAC3: A part of MEL1 (Liljestrom, P.L., Nucl. Acids Res., 13, pp.7257-7268, 1985) was amplified by PCR (template: IFO 0565 strain chromosomal DNA, primers: MEL1-sen1 and MEL1-anti1) and subcloned into pGEM-T.
pUAC5a: A fragment obtained by digesting pUAC3 with ScaI and BglII was inserted into the ScaI and BglII sites of pUAC1a.
pUAC8: A part of MEL1 was amplified by PCR (template: IFO 1226 strain chromosomal DNA, primers: MEL1-sen3 and MEL1-anti1) and subcloned into pGEM-T.
pUAC12: A part of SUC2 (Taussig, R. et al., Nucl. Acids Res., 11, pp.1943-1954, 1983) was amplified by PCR (template: YHP500 strain chromosomal DNA, primers: SUC2-sen1 and SUC2-anti2) and subcloned into pGEM-T.
pUAC13: A part of MEL1 was amplified by PCR (template: IFO 0565 strain chromosomal DNA, primers: MEL1-sen3 and MEL1-anti3) and subcloned into pGEM-T.
pUAC14: A mutation was introduced into pGFPuv (primers: GFP-SM2 and GFP-SM3). pUAC15: pEAC8a was digested with StuI and self-ligated.
pUAC15a: A mutation was introduced into pUAC15 (primers: YEX-SM4 and YEX-SM7).
pUAC16: A fragment obtained by digesting pUAC12 with NaeI and SpeI was inserted between the SpeI and StuI sites of pUAC15a.
pUAC19: A fragment obtained by digesting pUXS1 with EcoRV and PvuII was inserted into the EcoRV site of pEAC12.
PUAC19a: A mutation was introduced into pUAC19 (primers: GFP-SM4, YEX-SM8 and YEX-SM9).
pUAC20: A fragment obtained by digesting pUXS2 with EcoRV and PvuII was inserted into the EcoRV site of pEAC12.
pUAC20a: A mutation was introduced into pUAC20 (primers: GFP-SM4, YEX-SM8 and YEX-SM9).
pUAC21: A fragment obtained by digesting pYPR2831 with PstI was blunt-ended and inserted into the EcoRV site of pEAC12.
pUAC21a: A fragment obtained by digesting pUAC19a with SacI and BglII was inserted between the SacI and BglII sites of pUAC21.
pUAC28: A part of GAS1 (Vai, M., et al., J. Biol. Chem., 266, 12242-12248, 1990) was amplified by PCR (template: YPH500 strain chromosomal DNA, primers: GAS1-sen1 and GAS1-anti3) and subcloned into pGEM-T.
pUAC29b: A fragment obtained by digesting pUAC28 with BglII and SacII was inserted between the BglII and SacII sites of pUAC20a.
pUAC30b: A fragment obtained by digesting pUAC29b with BglII and BamHI was inserted into the BglII site of pUAC19a.
pUAC31b: A fragment obtained by digesting pUAC29b with BglII and BamHI was inserted into the BglII site of pUAC21a.
pEAC3: A fragment obtained by digesting pUAC5a with EcoRI and SalI was inserted between the EcoRI and Sail sites of pYPR2831.
pEAC6: A part of pEAC3 was amplified by PCR (template: pEAC3, primers: MEL1-sen3 and MEL1-anti2) and subcloned into pGEM-T, and a fragment obtained by digesting the obtained plasmid with SacI was inserted into the SacI site of pYEX-S1. pEAC6a: A mutation was introduced into pEAC6 (primers: YEX-SM2 and YEX-SM3). pEAC7: A fragment obtained by digesting pUAC8 with SacI and BglII was inserted between the SacI and BglII sites of pEAC6a.
pEAC7a: A mutation was introduced into pEAC7 (primers: YEX-SM4 and YEX-SM5).
pEAC8: A fragment obtained by digesting pUAC13 with SacI and BglII was inserted between the SacI and BglII sites of pEAC6a.
pEAC8a: A mutation was introduced into pEAC8 (primer: YEX-SM6).
pEAC9: A fragment obtained by digesting pUAC14 with SacI and BglII was inserted between the SacI and BglII sites of pEAC6a.
pEAC11: A fragment obtained by digesting pEAC7a with SacI and NdeI was inserted between the SacI and NdeI sites of pUAC16.
pEAC12: A fragment obtained by digesting pEAC9 with SacI and BglII was inserted into the SacI and BglII sites of pEAC11.

In Fig. 1, for pEAC6a, Reporter is *α* -galactosidase. For pEAC8a, Reporter is GFPuv. For pUAC19, Reporter is EGFP, Marker is URA3, and AS is CWP2 anchoring signal. For pUAC19a, Reporter is EGFP, Marker is URA3, and AS is CWP2 anchoring signal. For pUAC20a, Reporter is EGFP, Marker is LEU2, and AS is CWP2 anchoring signal. For pUAC21a, Reporter is EGFP, Marker is TRP1, and AS is CWP2 anchoring signal. For pUAC29b, Reporter is EGFP, Marker is LEU2, and AS is GAS1 anchoring signal. For pUAC30b, Reporter is EGFP, Marker is URA3, and AS is GAS1 anchoring signal. For pUAC31b, Reporter is EGFPuv, Marker is TRP1, and AS is GAS1 anchoring signal.

### (E) Preparation of arming yeast

As a reporter protein, α-galactosidase derived from *S. cerevisiae* (Turakainen, H., et al., Appl. Environ. Microbiol., 59, 2622-2630, 1993; MEL1) or a green fluorescent protein (GFP) was chosen. As GFP, GFPuv and EGFPuv (Cormack, B.P., et al., Gene, 173, 33-38, 1996; obtained by introducing a mutation into GFPuv so that Phe64 and Ser65 is replaced with Leu and Thr, respectively) were used. A gene coding for a fusion protein which consists of each of the proteins added with a secretory signal of SUC2 at the N-terminus and a GPI anchoring signal of CWP2 at the C-terminus was designed, and the gene was inserted between the promoter and the terminator of phosphoglycerate kinase gene to obtain an expression cassette. Wall-type arming yeasts were obtained by transformation using a plasmid obtained by inserting the expression cassette into a YEp-type vector (pEAC6a, pEAC8a or pEAC9) for AYE1, AYE2 or AYE3 strain, or by linearizing a plasmid obtained by inserting the expression cassette into a YIp type vector (pUAC19a, pUAC20a or pUAC21a) and then introducing into chromosomal DNA of each strain (AY-2, AY-5, AY-16, AY-14 or AY-17 strain). Membrane-type arming yeast (AY-12 or AY-15 strain) was prepared by replacing the GPI anchoring signal with any of those derived from GAS1 (pUAC29b, pUAC30b or pUAC31b). The prepared strains are shown in Table 2.

**Table 2**

| Yeast strain | Genotype | Phenotype | Gene manipulation |
|---|---|---|---|
| YPH500 (parent strain) | MAT *α* ade2, his3, leu2, lys2, trp1, ura3 | | |
| AYE-1 | MAT *α* YPH500 (pEAC6a) | Leu+, Ura+ | |
| AYE-2 | MAT *α* YPH500 (pEAC8a) | Leu+, Ura+ | |
| AYE-3 | MAT *α* YPH500 (pEAC9) | Leu+, Ura+ | |
| AY-2 | MAT *α* YPH500 | Ura+ | EGFPuv - CWP2 (pUAC19a) |
| AY-5 | MAT *α* YPH500 | Leu+, Ura+ | EGFPuv - CWP2 x 2(pUAC19a, pUAC20a) |
| AY-10 | MAT *α* YPH500 | Leu+, Trp+ | EGFPuv - CWP2(pUAC20a, pUAC21a) |
| AY-12 | MAT *α* YPH500 | Leu+, Ura+ | EGFPuv - GAS1 x 2(pUAC29b, pUAC31b) |
| AY-14 | MAT *α* YPH500 | Leu+, Trp+, Ura+ | EGFPuv - CWP2 x 3 (pUAC19a, pUAC20a. pUAC21a) |
| AY-15 | MAT *α* YPH500 | Leu+, Trp+, Ura+ | EGFPuv - GAS1 x 3 (pUAC29b, pUAC30b. pUAC31b) |
| AY-16 | MAT *α* YPH500 kre6Δ::URA3 | Leu+, Trp+, Ura+ | EGFPuv - CWP2 x 2(pUAC20a. pUAC21a) |
| AY-17 | MAT *α* YPH500 | Leu+, Ura+ | GFPuv - CWP2 x 2(pUAC19, pUAC20) |

### (F) Media

YPAUD (1% yeast extract, 2% peptone, 2% glucose, 40 *µ* g/ml of adenine, 20 *µ* g/ml of uracil), RPMIB (RPMI1640 (Sigma), 1 M sorbitol, 100 mM potassium phosphate buffer (pH 4.0-7.0), 2% glucose, 40 *µ* g/ml of adenine, 20 *µ* g/ml of uracil) and YNB (0.67% yeast nitrogen base without amino acid (Difco), 2% glucose, additional nutrients (40 *µ* g/ml of adenine, 20 *µ* g/ml of histidine, 60 *µ* g/ml of leucine, 30 *µ* g/ml of lysine, 40 *µ* g/ml of tryptophan, 20 *µ* g/ml of uracil)) were appropriately used. Agar media were obtained by adding 1.5-2% agarose to the aforementioned liquid media.

### (G) Determination of α -galactosidase activity

The method of Schreuder et al. (Schreuder, M.P., et al., Yeast, 9, 399-409, 1993) was used. 160 *µ* l of a culture broth of a yeast strain cultured in YNB in the late logarithmic growth phase was added with 20 µl each of 1 M acetate buffer (pH 4.5) and 0.1 M p-nitrophenyl-α-galactopyranoside (Boehringer Manheim) and allowed to react at 37°C for 5 minutes. The reaction mixture was added with 1 ml of 2% sodium carbonate, and then the absorbance (OD₄₁₀) was measured.

### (H) Measurement of fluorescence intensity of GFP-expressing strain

Yeast cells cultured in RPMIB (pH 7.0) in the logarithmic growth phase were collected and floated in water at OD₅₉₅ = 1.0, and measurement was performed. The fluorescence intensity and the optimum wavelengths were measured by using a fluorometer (F-2000, Hitachi Koki Co., Ltd.), Further, fluorescence of each cell was detected by using a fluorescence microscope (Axioplan, Zeiss).

### (I) Determination of GFP releasing effect by Zymolyase action

Yeast cells cultured in a liquid medium in the logarithmic growth phase were collected and floated in an appropriate buffer. The mixture was added with 400-6.25 *µ* g/ml of Zymolyase 100T (Seikagaku Corporation) and shaken at 30°C for 30 minutes. After the reaction, the yeast cells and the buffer were separated by filtration through a filter, and fluorescence intensity in the buffer was measured by a fluorometer (excitation = 487 nm, emission = 513 nm).

### (J) Comparison of GFP localization

Yeast cells (AY-2 strain) cultured in a liquid medium in the logarithmic growth phase were physically disrupted by using glass beads, then the cell wall, cell membrane and soluble proteins were fractionated, and each fraction was suspended in an appropriate buffer. The fluorescence intensity of each fraction was measured (excitation = 487 nm, emission = 513 nm) and represented in terms of a ratio based on the total fluorescence intensity of the whole cells.

### (K) Determination of GFP releasing effect in KRE6-disrupted strain

The KRE6-disrupted strain (AY-16 strain) was cultured with shaking at 30°C. The culture fluid was filtered using a filter after 3 and 6 hours, and fluorescence intensity in the medium was measured.

### (L) Measurement of GFP releasing effect of available antifungal agents

The cultured yeast cells in the logarithmic growth phase (AY-2 strain or AY-12 strain) were collected, and various agents were allowed to act on the yeast cells. Fluorescence intensity of the culture supernatant was measured by using Cytofluor 2300 Fluorometer (Millipore, excitation = 480 nm, emission = 530 nm). The fluorescence intensity under treatment with each of various agents was shown in terms of a difference in fluorescence intensity compared with that of a control in which no agent was added.

### (2) Results

### (A) Characterization of various wall-type arming yeast

Various wall-type arming yeasts were produced by using three kinds of reporter proteins, α-galactocidase, GFPuv and EGFPuv, and applying a method of using a high copy number vector or a method of insertion into the chromosome as an expression method, and α-galactosidase activity and fluorescence intensity given by these yeast strains were compared. As a result, only weak activity was observed in both of the α-galactosidase-expressing strains (AYE-1 strain and AYE-2 strain). Further, when a yeast strain (AYE-3 strain) in which GFPuv was expressed by using a high copy number vector was examined with a fluorescence microscope, significant scattering was observed in the fluorescence intensity among individual cells, which suggested that there was a problem in stability of the plasmid. Whilst when fluorescence intensities of the strains obtained by inserting EGFPuv and GFPuv expression cassettes into the chromosome (AY-5 strain and AY-17 strain) were compared (Table 3), the fluorescence intensity of EGFPuv was three or more times stronger than that of GFPuv. Based on these results, it was concluded that use of a fluorescent protein as the reporter protein was appropriate and that the strain in which EGFPuv was inserted into the chromosome as the reporter protein was particularly preferred. When the optimum wavelengths of the strain were measured, excitation max was found as 487 nm, and emission max as 513 nm.

**Table 3**

| | Excitation (nm) | Emission (nm) | Fluorescence |
|---|---|---|---|
| YPH500 | 487 | 513 | 260 |
| AY-5 | 487 | 513 | 1260 |
| YPH500 | 395 | 509 | 50 |
| AY-17 | 395 | 509 | 350 |

| | | | |
|---|---|---|---|
| The strain in the logarithmic growth phase was suspended in water and its fluorescence intensity was measured. | | | |

### (B) Measurement of GFP releasing effect by Zymolyase action

The AY-2 strain was grown in a test tube, and the cells were collected and Zymolyase was allowed to act on the cells. The fluorescence intensity in the culture supernatant after subjected to the action was measured. When Zymolyase was allowed to act on the AY-2 strain under osmotic pressure protection, the fluorescence intensity in the buffer increased depending on the concentration of the added Zymolyase (Table 4). These results suggested that a large amount of GFP was fixed on the cell wall.

**Table 4**

| Zymolyase (µg/ml) | Fluorescence intensity |
|---|---|
| 400 | 2160 |
| 200 | 1240 |
| 100 | 1170 |
| 50 | 786 |
| 25 | 410 |
| 12.5 | 215 |
| 6.25 | 126 |
| 0 | 107 |

### (C) Measurement of GFP releasing effect in KRE6-disrupted strain

It is estimated that at least 6 kinds of enzymes are involved in the biosynthesis of (1,6)-β-glucan in *Saccharomyces cerevisiae*. Among these enzymes, it has been revealed that at least one of a product (a protein) encoded by the KRE6- gene present in the Golgi body and a product (a protein) encoded by SKN1 gene, which is a homologue thereof, is essential for the growth (Gaughran, J.P. et al., J. Bacteriol., 176, pp.5857-5860, 1994). Further, it is estimated that KRE6 homologues widely exist also in fungi such as *Candida albicans* (typical pathogenic fungus). Based on the above findings, Kre6p (a KRE6 gene product) is expected to be a preferred target for development of novel antifungal agents.

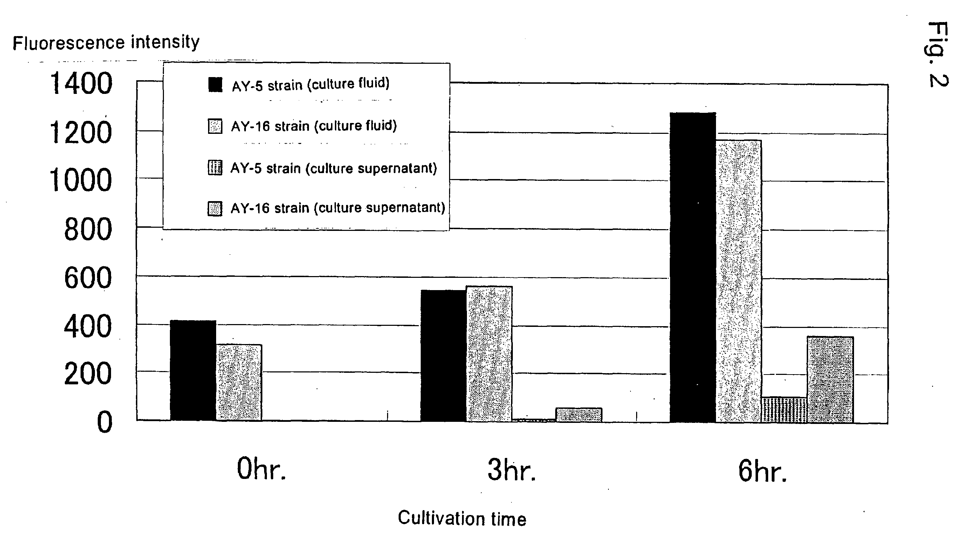

In order to verify the GFP releasing effect by the disruption of KRE6, the AY-5 strain and the AY-16 (KRE6-disrupted) strain were cultured in test tubes, and release of GFP with passage of time was measured (Fig. 2). As a result, almost same level of fluorescence intensities were detected in the culture fluids (bacteria + medium) for the both strains at any time during the cultivation period, whilst the fluorescence intensity in the culture supernatant of the AY-16 strain was apparently higher than that of the AY-5 strain. Further, when these two yeast strains were cultured in test tubes and cells in the logarithmic growth phase were examined under a fluorescence microscope (×400), the fluorescence intensity of the AY-16 strain was attenuated. These results suggest that disruption of KRE6 accelerates GFP release. Accordingly, these experiment results indicate that an agent inhibiting the KRE6 gene product can be successfully screened by using the microorganisms prepared (arming yeasts).

### (D) Comparison of GFP localization

Localization of GFP was compared in the AY-2 strain (wall-type arming yeast) and the AY-12 strain (membrane-type arming yeast). Each yeast strain was grown in a test tube, and cells were collected and fractionated into cell wall, cell membrane, and soluble proteins. Fluorescence intensities of the resulting fractions were measured and each ratio on the basis of the total fluorescence intensity was calculated (unit: %). The results are shown in Table 5. It was revealed that a respective large amount of GFP was fixed on the cell wall of the wall-type arming yeast and the cell membrane of the membrane-type arming yeast.

**Table 5**

| | AY-2 | AY-12 |
|---|---|---|
| Cell wall fraction | 40 | 11 |
| Cell membrane fraction | 15 | 48 |
| Soluble protein fraction | 45 | 41 |

### (E) Measurement of GFP releasing effect of available antifungal agents

In order to ascertain whether or not the method of the present invention can be utilized for a screening of agents with a targeting site other than Kre6p, the GFP releasing effects in the AY-2 strain (wall-type arming yeast) and the AY-12 strain (membrane-type arming yeast) were compared under actions of available antifungal agents. The AY-2 strain cells and the AY-12 strain cells each cultured in a test tube were floated on a medium (RPMIB) protected for osmotic pressure, and each of the agents was allowed to act on the cells. After the culture, the fluorescence intensity of the supernatant was measured, and a difference from that of a control with no addition of the agent was calculated. The results are shown in Fig. 3.

Marked GFP releasing effect was observed in the AY-2 strain treated with Aculeacin A ((1,3)-β-glucan synthesis inhibitor) and Tunicamycin (mannan synthesis inhibitor) both acting on cell walls, whereas almost no releasing effect was observed in the AY-12 strain. Slight releasing effect was observed in the both strains treated with Amphotericin B, Fluconazole and Salinomycin which act on cell membrane. Agents other than the above gave no clear GFP releasing effect only on the wall-type arming yeast (AY-2 strain). These results revealed that various kinds of agent acting on cell wall can be successfully screened by the screening method of the present invention.

The genes incorporated in the AY-15 strain and the AY-14 strain, which are the microorganisms of the present invention, are shown in the following Sequence Listing shows.

SEQ ID NO: 1 is the gene incorporated in the AY-15 strain, which is a membrane-type arming yeast, as a genome-incorporated type gene. The gene corresponds to a nucleotide sequence of SS (secretory signal)-EGFPuv-membrane anchoring signal (AS) and was obtained by adding mutations to a commercially available GFPuv (312th position was substituted with "g", 315th position with "a", and 316th position with "a" for the nucleotides in the original GFPuv being t, c and t, respectively).

SEQ ID NO: 2 is the gene incorporated in the AY-14 strain, which is a wall-type arming yeast, as a genome-incorporated type gene. The gene corresponds to a nucleotide sequence of SS (secretory signal)-EGFPuv-wall anchoring signal (AS) and was obtained by adding mutations to a commercially available GFPuv (312th position was replaced with "g", 315th position with "a" and 316th position with "a" for the nucleotides in the original GFPuv being t, c and t, respectively).

### Industrial Applicability

According to the present invention, agents having a selective inhibitory action on cell walls, for example, agents having a selective inhibitory action on an enzyme for biosynthesis of (1,6)-β-glucan that constitutes cell walls can be efficiently screened.

## Claims

1. A method for screening an agent acting on a cell wall, which comprises the steps of:
(1) culturing each of a microorganism having a reporter protein fixed on a cell wall as a GPI-anchored protein and a microorganism having a reporter protein fixed on a cell membrane as a GPI-anchored protein in the presence of a test agent;
(2) determining the reporter protein released into each culture fluid of the microorganism; and
(3) judging that the test agent has a selective inhibitory action on the cell wall when the reporter protein is released from the microorganism having the reporter protein fixed on the cell wall into the culture fluid and the reporter protein is not substantially released from the microorganism having the reporter protein fixed on the cell membrane into the culture fluid.

2. The method according to claim 1, which uses a microorganism having the reporter protein fixed to (1,6)-β-glucan on the cell wall.

3. The method according to claim 1 or 2, wherein the microorganism is yeast.

4. The method according to any one of claims 1 to 3, wherein the reporter protein is a fluorescent protein.

5. The method according to claim 4, wherein the fluorescent protein is a green fluorescent protein.

6. The method according to any one of claims 1 to 5, wherein the inhibitory action on the cell wall is an inhibitory action on a biosynthetic process and/or an enzyme for biosynthesis of the cell wall.

7. The method according to any one of claims 1 to 6, wherein the agent acting on the cell wall is an antifungal agent.

8. Use of a transformed microorganism having a reporter protein fixed on a cell wall as a GPI-anchored protein, which reporter protein comprised in said microorganism is recombinant, preferably as fusion protein, in the method for screening an agent acting on a cell wall according to any one of claims 1 to 7.

9. The use according to claim 8, wherein the reporter protein is fixed to (1,6)- β - glucan of the cell wall.

10. The use according to claims 8 or 9, which is used for a screening of an agent acting on a cell wall.

11. The use according to claim 10, wherein the agent acting on a cell wall is an antifungal agent.

12. The use according to any one of claims 8 to 11, wherein the microorganism is an yeast.

13. The useaccording to any one of claims 8 to 12, wherein the reporter protein is a fluorescent protein.

14. The use according to claim 13, wherein the fluorescent protein is a green fluorescent protein.

15. Use of a set of microorganisms in the method for screening an agent acting on a cell wall according to any one of claims 1 to 7, wherein the set of microorganisms comprises:
(1) a transformed microorganism having a reporter protein fixed on a cell wall as a GPI-anchored protein, which reporter protein comprised in said microorganism is recombinant, preferably as fusion protein; and
(2) a transformed microorganism having a reporter protein fixed on a cell membrane as a GPI-anchored protein, which reporter protein comprised in said microorganism is recombinant, preferably as fusion protein.

16. The useaccording to claim 15, which comprises a microorganism having a reporter protein fixed to (1,6)- β -glucan of the cell wall.

17. The use according to claim 15 or 16, wherein the agent acting on a cell wall is an antifungal agent.

18. The use according to any one of claims 15 to 17, wherein the microorganisms are yeasts.

19. The use according to any one of claims 15 to 18, wherein the reporter protein is a fluorescent protein.

20. The use according to claim 19, wherein the fluorescent protein is a green fluorescent protein.

## Patentansprüche

1. Verfahren zum Screening eines auf eine Zellwand wirkenden Mittels, umfassend die Schritte:
(1) Kultivieren jeweils eines Mikroorganismus, der ein an einer Zellwand fixiertes Reporterprotein als GPI-verankertes Protein aufweist, und eines Mikroorganismus, der ein an einer Zellmembran fixiertes Reporterprotein als GPI-verankertes Protein aufweist, in Gegenwart eines Testmittels;
(2) Bestimmen des Reporterproteins, das in die jeweilige Kulturflüssigkeit des Mikroorganismus freigesetzt wird; und
(3) Beurteilen, dass das Testmittel eine selektive hemmende Wirkung auf die Zellwand aufweist, wenn das Reporterprotein von dem Mikroorganismus mit dem an der Zellwand fixierten Reporterprotein in die Kulturflüssigkeit freigesetzt und das Reporterprotein von dem Mikroorganismus mit dem an der Zellmembran fixierten Reporterprotein im Wesentlichen nicht in die Kulturflüssigkeit freigesetzt wird.

2. Verfahren nach Anspruch 1, wobei ein Mikroorganismus verwendet wird, bei dem das Reporterprotein an β-1,6-Glucan an der Zellwand fixiert ist.

3. Verfahren nach Anspruch 1 oder 2, wobei der Mikroorganismus eine Hefe ist.

4. Verfahren nach einem der Ansprüche 1 bis 3, wobei das Reporterprotein ein fluoreszierendes Protein ist.

5. Verfahren nach Anspruch 4, wobei das fluoreszierende Protein ein grün fluoreszierendes Protein ist.

6. Verfahren nach einem der Ansprüche 1 bis 5, wobei die hemmende Wirkung auf die Zellwand eine hemmende Wirkung auf einen Biosynthese-Vorgang und/oder ein Enzym für die Biosynthese der Zellwand ist.

7. Verfahren nach einem der Ansprüche 1 bis 6, wobei das auf die Zellwand wirkende Mittel ein antifungales Mittel ist.

8. Verwendung eines transformierten Mikroorganismus, der ein an einer Zellwand fixiertes Reporterprotein als GPI-verankertes Protein aufweist, wobei das in dem Mikroorganismus enthaltene Reporterprotein rekombinant ist, vorzugsweise als Fusionsprotein, bei dem Verfahren zum Screening eines auf eine Zellwand wirkenden Mittels nach einem der Ansprüche 1 bis 7.

9. Verwendung nach Anspruch 8, wobei das Reporterprotein an β-1,6-Glucan der Zellwand fixiert ist.

10. Verwendung nach Anspruch 8 oder 9 zum Screening eines auf eine Zellwand wirkenden Mittels.

11. Verwendung nach Anspruch 10, wobei das auf eine Zellwand wirkende Mittel ein antifungales Mittel ist.

12. Verwendung nach einem der Ansprüche 8 bis 11, wobei der Mikroorganismus eine Hefe ist.

13. Verwendung nach einem der Ansprüche 8 bis 12, wobei das Reporterprotein ein fluoreszierendes Protein ist.

14. Verwendung nach Anspruch 13, wobei das fluoreszierende Protein ein grün fluoreszierendes Protein ist.

15. Verwendung eines Satzes von Mikroorganismen bei dem Verfahren zum Screening eines auf eine Zellwand wirkenden Mittels nach einem der Ansprüche 1 bis 7, wobei der Mikroorganismen-Satz folgendes umfasst:
(1) einen transformierten Mikroorganismus, der ein an einer Zellwand fixiertes Reporterprotein als GPI-verankertes Protein aufweist, wobei das in dem Mikroorganismus enthaltene Reporterprotein rekombinant ist, vorzugsweise als Fusionsprotein; und
(2) einen transformierten Mikroorganismus, der ein an einer Zellmembran fixiertes Reporterprotein als GPI-verankertes Protein aufweist, wobei das in dem Mikroorganismus enthaltene Reporterprotein rekombinant ist, vorzugsweise als Fusionsprotein.

16. Verwendung nach Anspruch 15, umfassend einen Mikroorganismus, bei dem ein Reporterprotein an β-1,6-Glucan der Zellwand fixiert ist.

17. Verwendung nach Anspruch 15 oder 16, wobei das auf eine Zellwand wirkende Mittel ein antifungales Mittel ist.

18. Verwendung nach einem der Ansprüche 15 bis 17, wobei die Mikroorganismen Hefen sind.

19. Verwendung nach einem der Ansprüche 15 bis 18, wobei das Reporterprotein ein fluoreszierendes Protein ist.

20. Verwendung nach Anspruch 19, wobei das fluoreszierende Protein ein grün fluoreszierendes Protein ist.

## Revendications

1. Procédé de criblage d'un agent agissant sur une paroi cellulaire, qui comprend les étapes consistant à :
(1) mettre en culture chacun d'un micro-organisme ayant une protéine marqueur fixée sur une paroi cellulaire en tant que protéine à ancre GPI et d'un micro-organisme ayant une protéine marqueur fixée sur une membrane cellulaire en tant que protéine à ancre GPI en présence d'un agent d'essai ;
(2) déterminer la protéine marqueur libérée dans chaque fluide de culture du micro-organisme ; et
(3) juger que l'agent d'essai a une action inhibitrice sélective sur la paroi cellulaire lorsque la protéine marqueur est libérée du micro-organisme ayant la protéine marqueur fixée sur la paroi cellulaire dans le fluide de culture et que la protéine marqueur n'est pas sensiblement libérée du micro-organisme ayant la protéine marqueur fixée sur la membrane cellulaire dans le fluide de culture.

2. Procédé selon la revendication 1, qui utilise un micro-organisme ayant la protéine marqueur fixée au (1,6)-β-glucane sur la paroi cellulaire.

3. Procédé selon la revendication 1 ou 2, dans lequel le micro-organisme est une levure.

4. Procédé selon l'une quelconque des revendications 1 à 3, dans lequel la protéine marqueur est une protéine fluorescente.

5. Procédé selon la revendication 4, dans lequel la protéine fluorescente est une protéine fluorescente verte.

6. Procédé selon l'une quelconque des revendications 1 à 5, dans lequel l'action inhibitrice sur la paroi cellulaire est une action inhibitrice sur un procédé biosynthétique et/ou une enzyme de biosynthèse de la paroi cellulaire.

7. Procédé selon l'une quelconque des revendications 1 à 6, dans lequel l'agent agissant sur la paroi cellulaire est un fongicide.

8. Utilisation d'un micro-organisme transformé ayant une protéine marqueur fixée sur une paroi cellulaire en tant que protéine à ancre GPI, laquelle protéine marqueur comprise dans ledit micro-organisme est recombinée, de préférence en tant que protéine hybride, dans le procédé de criblage d'un agent agissant sur une paroi cellulaire selon l'une quelconque des revendications 1 à 7.

9. Utilisation selon la revendication 8, dans laquelle la protéine marqueur est fixée au (1,6)-β-glucane de la paroi cellulaire.

10. Utilisation selon la revendication 8 ou 9, qui est utilisée pour le criblage d'un agent agissant sur une paroi cellulaire.

11. Utilisation selon la revendication 10, dans laquelle l'agent agissant sur une paroi cellulaire est un fongicide.

12. Utilisation selon l'une quelconque des revendications 8 à 11, dans laquelle le micro-organisme est une levure.

13. Utilisation selon l'une quelconque des revendications 8 à 12, dans laquelle la protéine marqueur est une protéine fluorescente.

14. Utilisation selon la revendication 13, dans laquelle la protéine fluorescente est une protéine fluorescente verte.

15. Utilisation d'un ensemble de micro-organismes dans le procédé de criblage d'un agent agissant sur une paroi cellulaire selon l'une quelconque des revendications 1 à 7, dans laquelle l'ensemble de micro-organismes comprend :
(1) un micro-organisme transformé ayant une protéine marqueur fixée sur une paroi cellulaire en tant que protéine à ancre GPI, laquelle protéine marqueur comprise dans ledit micro-organisme est recombinée, de préférence en tant que protéine hybride ; et
(2) un micro-organisme transformé ayant une protéine marqueur fixée sur une membrane cellulaire en tant que protéine à ancre GPI, laquelle protéine marqueur comprise dans ledit micro-organisme est recombinée, de préférence en tant que protéine hybride.

16. Utilisation selon la revendication 15, qui comprend un micro-organisme ayant une protéine marqueur fixée au (1,6)-β-glucane de la paroi cellulaire.

17. Utilisation selon la revendication 15 ou 16, dans lequel l'agent agissant sur une paroi cellulaire est un fongicide.

18. Utilisation selon l'une quelconque des revendications 15 à 17, dans laquelle les micro-organismes sont des levures.

19. Utilisation selon l'une quelconque des revendications 15 à 18, dans laquelle la protéine marqueur est une protéine fluorescente.

20. Utilisation selon la revendication 19, dans laquelle la protéine fluorescente est une protéine fluorescente verte.


### SEQUENCE LISTING

### Sequence Listing

<110> Daiichi Pharmaceutical Co., Ltd.
   <120> Method for screening an agent acting on cell wall
   <130> A11039M
   <160> 2
<210> 1
   <211> 1116
   <212> DNA
   <213> Saccharomyces cerevisiae
<400> 1
   actagtatgc ttttgcaagc tttccttttc cttttggctg gttttgcagc caaaatatct 60
   gcatcaaaga gctcgcatgc ctgcaggtcg actctagagg atccccgggt accggtagaa 120
   aaaatgagta aaggagaaga acttttcact ggagttgtcc caattcttgt tgaattagat 180
   ggtgatgtta atgggcacaa attttctgtc agtggagagg gtgaaggtga tgcaacatac 240
   ggaaaactta cccttaaatt tatttgcact actggaaaac tacctgttcc atggccaaca 300
   cttgtcacta cgttaactta tggtgttcaa tgcttttccc gttatccgga tcatatgaaa 360
   cggcatgact ttttcaagag tgccatgccc gaaggttatg tacaggaacg cactatatct 420
   ttcaaagatg acgggaacta caagacgcgt gctgaagtca agtttgaagg tgataccctt 480
   gttaatcgta tcgagttaaa aggtattgat tttaaagaag atggaaacat tctcggacac 540
   aaactcgagt acaactataa ctcacacaat gtatacatca cggcagacaa acaaaagaat 600
   ggaatcaaag ctaacttcaa aattcgccac aacattgaag atggatccgt tcaactagca 660
   gaccattatc aacaaaatac tccaattggc gatggccctg tccttttacc agacaaccat 720
   tacctgtcga cacaatctgc cctttcgaaa gatcccaacg aaaagcgtga ccacatggtc 780
   cttcttgagt ttgtaactgc tgctgggatt acacatggca tggatgagat ctgtagtgtt 840
   gatttgggtt ccggaactga atccagtact gcctcttcta acgcttcggg gtcttcttcc 900
   aagtctaact ccggctcttc tggttcttcc agttcttctt cttcttcttc agcttcatct 960
   tcatcttcta gcaagaagaa tgctgccacc aacgttaaag ctaacttagc acaagtggtc 1020
   tttacctcca tcatttcctt atccattgcc gctggtgtcg gttttgcttt ggtttaaaaa 1080
   gcttcgacac atacataata actcgataag ccgcgg 1116
<210> 2
   <211> 1236
   <212> DNA
   <213> Saccharomyces cerevisiae
<400> 2
   actagtatgc ttttgcaagc tttccttttc cttttggctg gttttgcagc caaaatatct 60
   gcatcaaaga gctcgcatgc ctgcaggtcg actctagagg atccccgggt accggtagaa 120
   aaaatgagta aaggagaaga acttttcact ggagttgtcc caattcttgt tgaattagat 180
   ggtgatgtta atgggcacaa attttctgtc agtggagagg gtgaaggtga tgcaacatac 240
   ggaaaactta cccttaaatt tatttgcact actggaaaac tacctgttcc atggccaaca 300
   cttgtcacta cgttaactta tggtgttcaa tgcttttccc gttatccgga tcatatgaaa 360
   cggcatgact ttttcaagag tgccatgccc gaaggttatg tacaggaacg cactatatct 420
   ttcaaagatg acgggaacta caagacgcgt gctgaagtca agtttgaagg tgataccctt 480
   gttaatcgta tcgagttaaa aggtattgat tttaaagaag atggaaacat tctcggacac 540
   aaactcgagt acaactataa ctcacacaat gtatacatca cggcagacaa acaaaagaat 600
   ggaatcaaag ctaacttcaa aattcgccac aacattgaag atggatccgt tcaactagca 660
   gaccattatc aacaaaatac tccaattggc gatggccctg tccttttacc agacaaccat 720
   tacctgtcga cacaatctgc cctttcgaaa gatcccaacg aaaagcgtga ccacatggtc 780
   cttcttgagt ttgtaactgc tgctgggatt acacatggca tggatgagat ctctgaatcc 840
   gctgccgcca tttctcaaat cactgacggt caaatccaag ctactaccac tgctaccacc 900
   gaagctacca ccactgctgc cccatcttcc accgttgaaa ctgtttctcc atccagcacc 960
   gaaactatct ctcaacaaac tgaaaatggt gctgctaagg ccgctgtcgg tatgggtgcc 1020
   ggtgctctag ctgctgctgc tatgttgtta taagaaatct ctgatttttt ataatatcta 1080
   tatggctttt ttcaaaattt tcggttttac taggtaagtg tttgattctt ttcttttcgt 1140
   taatatattt ttacacataa ttttaaataa tttttgttat tttgaatagg tagataccat 1200
   aaaaataaaa cactttttac tttaacgagt ccgcgg 1236

### SEQUENCE LISTING

<110> Daiichi Pharmaceutical Co., Ltd.
   <120> METHOD FOR SCREENING AGENT ACTING ON CELL WALL
   <130> P20599EP
   <140> EP 01 925 961.3
   <141> 2001-04-26
   <150> JP 132041/2000
   <151> 2000-05-01
   <160> 24
   <170> PatentIn Ver. 2.1
<210> 1
   <211> 1116
   <212> DNA
   <213> Saccharomyces cerevisiae
<400> 1
   actagtatgc ttttgcaagc tttccttttc cttttggctg gttttgcagc caaaatatct 60
   gcatcaaaga gctcgcatgc ctgcaggtcg actctagagg atccccgggt accggtagaa 120
   aaaatgagta aaggagaaga acttttcact ggagttgtcc caattcttgt tgaattagat 180
   ggtgatgtta atgggcacaa attttctgtc agtggagagg gtgaaggtga tgcaacatac 240
   ggaaaactta cccttaaatt tatttgcact actggaaaac tacctgttcc atggccaaca 300
   cttgtcacta cgttaactta tggtgttcaa tgcttttccc gttatccgga tcatatgaaa 360
   cggcatgact ttttcaagag tgccatgccc gaaggttatg tacaggaacg cactatatct 420
   ttcaaagatg acgggaacta caagacgcgt gctgaagtca agtttgaagg tgataccctt 480
   gttaatcgta tcgagttaaa aggtattgat tttaaagaag atggaaacat tctcggacac 540
   aaactcgagt acaactataa ctcacacaat gtatacatca cggcagacaa acaaaagaat 600
   ggaatcaaag ctaacttcaa aattcgccac aacattgaag atggatccgt tcaactagca 660
   gaccattatc aacaaaatac tccaattggc gatggccctg tccttttacc agacaaccat 720
   tacctgtcga cacaatctgc cctttcgaaa gatcccaacg aaaagcgtga ccacatggtc 780
   cttcttgagt ttgtaactgc tgctgggatt acacatggca tggatgagat ctgtagtgtt 840
   gatttgggtt ccggaactga atccagtact gcctcttcta acgcttcggg gtcttcttcc 900
   aagtctaact ccggctcttc tggttcttcc agttcttctt cttcttcttc agcttcatct 960
   tcatcttcta gcaagaagaa tgctgccacc aacgttaaag ctaacttagc acaagtggtc 1020
   tttacctcca tcatttcctt atccattgcc gctggtgtcg gttttgcttt ggtttaaaaa 1080
   gcttcgacac atacataata actcgataag ccgcgg                           1116
<210> 2
   <211> 1236
   <212> DNA
   <213> Saccharomyces cerevisiae
<400> 2
   actagtatgc ttttgcaagc tttccttttc cttttggctg gttttgcagc caaaatatct 60
   gcatcaaaga gctcgcatgc ctgcaggtcg actctagagg atccccgggt accggtagaa 120
   aaaatgagta aaggagaaga acttttcact ggagttgtcc caattcttgt tgaattagat 180
   ggtgatgtta atgggcacaa attttctgtc agtggagagg gtgaaggtga tgcaacatac 240
   ggaaaactta cccttaaatt tatttgcact actggaaaac tacctgttcc atggccaaca 300
   cttgtcacta cgttaactta tggtgttcaa tgcttttccc gttatccgga tcatatgaaa 360
   cggcatgact ttttcaagag tgccatgccc gaaggttatg tacaggaacg cactatatct 420
   ttcaaagatg acgggaacta caagacgcgt gctgaagtca agtttgaagg tgataccctt 480
   gttaatcgta tcgagttaaa aggtattgat tttaaagaag atggaaacat tctcggacac 540
   aaactcgagt acaactataa ctcacacaat gtatacatca cggcagacaa acaaaagaat 600
   ggaatcaaag ctaacttcaa aattcgccac aacattgaag atggatccgt tcaactagca 660
   gaccattatc aacaaaatac tccaattggc gatggccctg tccttttacc agacaaccat 720
   tacctgtcga cacaatctgc cctttcgaaa gatcccaacg aaaagcgtga ccacatggtc 780
   cttcttgagt ttgtaactgc tgctgggatt acacatggca tggatgagat ctctgaatcc 840
   gctgccgcca tttctcaaat cactgacggt caaatccaag ctactaccac tgctaccacc 900
   gaagctacca ccactgctgc cccatcttcc accgttgaaa ctgtttctcc atccagcacc 960
   gaaactatct ctcaacaaac tgaaaatggt gctgctaagg ccgctgtcgg tatgggtgcc 1020
   ggtgctctag ctgctgctgc tatgttgtta taagaaatct ctgatttttt ataatatcta 1080
   tatggctttt ttcaaaattt tcggttttac taggtaagtg tttgattctt ttcttttcgt 1140
   taatatattt ttacacataa ttttaaataa tttttgttat tttgaatagg tagataccat 1200
   aaaaataaaa cactttttac tttaacgagt ccgcgg                           1236
<210> 3
   <211> 26
   <212> DNA
   <213> Artificial Sequence
<220>
   <223> Description of Artificial Sequence: Primer
<400> 3
   ggcatggatg agatctacaa ataatg 26
<210> 4
   <211> 27
   <212> DNA
   <213> Artificial Sequence
<220>
   <223> Description of Artificial Sequence: Primer
<400> 4
   catgattacg ccgagctcgc atgcctg 27
<210> 5
   <211> 37
   <212> DNA
   <213> Artificial Sequence
<220>
   <223> Description of Artificial Sequence: Primer
<400> 5
   caacacttgt cactacgtta acttatggtg ttcaatg 37
<210> 6
   <211> 25
   <212> DNA
   <213> Artificial Sequence
<220>
   <223> Description of Artificial Sequence: Primer
<400> 6
   cctgtgattt ctccagctcg aattc 25
<210> 7
   <211> 29
   <212> DNA
   <213> Artificial Sequence
<220>
   <223> Description of Artificial Sequence: Primer
<400> 7
   gattcattaa tgcatgctgg cacgacagg 29
<210> 8
   <211> 25
   <212> DNA
   <213> Artificial Sequence
<220>
   <223> Description of Artificial Sequence: Primer
<400> 8
   gattcattaa tgcagctggc acgac 25
<210> 9
   <211> 32
   <212> DNA
   <213> Artificial Sequence
<220>
   <223> Description of Artificial Sequence: Primer
<400> 9
   ctcacggtat cgccctcgag atctctgaat cc 32
<210> 10
   <211> 28
   <212> DNA
   <213> Artificial Sequence
<220>
   <223> Description of Artificial Sequence: Primer
<400> 10
   gagaccctct tctgagctct ctgaatcc 28
<210> 11
   <211> 34
   <212> DNA
   <213> Artificial Sequence
<220>
   <223> Description of Artificial Sequence: Primer
<400> 11
   aaaccaaaag atcgactagt ataaaatgaa tata 34
<210> 12
   <211> 22
   <212> DNA
   <213> Artificial Sequence
<220>
   <223> Description of Artificial Sequence: Primer
<400> 12
   cattaatgca tgctggcacg ac 22
<210> 13
   <211> 31
   <212> DNA
   <213> Artificial Sequence
<220>
   <223> Description of Artificial Sequence: Primer
<400> 13
   ctttaacgag tccgcggatt tctccagctc g 31
<210> 14
   <211> 32
   <212> DNA
   <213> Artificial Sequence
<220>
   <223> Description of Artificial Sequence: Primer
<400> 14
   gcactagtat gcttttgcaa gctttccttt tc 32
<210> 15
   <211> 33
   <212> DNA
   <213> Artificial Sequence
<220>
   <223> Description of Artificial Sequence: Primer
<400> 15
   gcgagctctt tgatgcagat attttggctg caa 33
<210> 16
   <211> 30
   <212> DNA
   <213> Artificial Sequence
<220>
   <223> Description of Artificial Sequence: Primer
<400> 16
   gcagatctgt agtgttgatt tgggttccgg 30
<210> 17
   <211> 38
   <212> DNA
   <213> Artificial Sequence
<220>
   <223> Description of Artificial Sequence: Primer
<400> 17
   gcccgcggct tatcgagtta ttatgtatgt gtcgaagc 38
<210> 18
   <211> 29
   <212> DNA
   <213> Artificial Sequence
<220>
   <223> Description of Artificial Sequence: Primer
<400> 18
   gcagatctac tttgttgccg ctgaatccg 29
<210> 19
   <211> 32
   <212> DNA
   <213> Artificial Sequence
<220>
   <223> Description of Artificial Sequence: Primer
<400> 19
   gcgaattcga gaaatcacag gactcgttaa ag 32
<210> 20
   <211> 36
   <212> DNA
   <213> Artificial Sequence
<220>
   <223> Description of Artificial Sequence: Primer
<400> 20
   gcgaattcga gagcaacggt aataaaagca acgacg 36
<210> 21
   <211> 30
   <212> DNA
   <213> Artificial Sequence
<220>
   <223> Description of Artificial Sequence: Primer
<400> 21
   cggagctcgg tgtctccgag ttacaatggc 30
<210> 22
   <211> 33
   <212> DNA
   <213> Artificial Sequence
<220>
   <223> Description of Artificial Sequence: Primer
<400> 22
   gcagatctag aaggcgatac cgtgagctgg aac 33
<210> 23
   <211> 30
   <212> DNA
   <213> Artificial Sequence
<220>
   <223> Description of Artificial Sequence: Primer
<400> 23
   cggagctcca tcaatacttc tcgccctgct 30
<210> 24
   <211> 34
   <212> DNA
   <213> Artificial Sequence
<220>
   <223> Description of Artificial Sequence: Primer
<400> 24
   gcagatctaa gaagagggtc tcaacctata gaag 34

### SEQUENCE LISTING

<110> Daiichi Pharmaceutical Co., Ltd.
   <120> METHOD FOR SCREENING AGENT ACTING ON CELL WALL
   <130> P20599EP
   <140> 01 925 961.3
   <141> 2001-04-26
   <150> JP 132041/2000
   <151> 2000-05-01
   <160> 2
   <170> PatentIn Ver. 2.1
<210> 1
   <211> 1116
   <212> DNA
   <213> Saccharomyces cerevisiae
<400> 1
   actagtatgc ttttgcaagc tttccttttc cttttggctg gttttgcagc caaaatatct 60
   gcatcaaaga gctcgcatgc ctgcaggtcg actctagagg atccccgggt accggtagaa 120
   aaaatgagta aaggagaaga acttttcact ggagttgtcc caattcttgt tgaattagat 180
   ggtgatgtta atgggcacaa attttctgtc agtggagagg gtgaaggtga tgcaacatac 240
   ggaaaactta cccttaaatt tatttgcact actggaaaac tacctgttcc atggccaaca 300
   cttgtcacta cgttaactta tggtgttcaa tgcttttccc gttatccgga tcatatgaaa 360
   cggcatgact ttttcaagag tgccatgccc gaaggttatg tacaggaacg cactatatct 420
   ttcaaagatg acgggaacta caagacgcgt gctgaagtca agtttgaagg tgataccctt 480
   gttaatcgta tcgagttaaa aggtattgat tttaaagaag atggaaacat tctcggacac 540
   aaactcgagt acaactataa ctcacacaat gtatacatca cggcagacaa acaaaagaat 600
   ggaatcaaag ctaacttcaa aattcgccac aacattgaag atggatccgt tcaactagca 660
   gaccattatc aacaaaatac tccaattggc gatggccctg tccttttacc agacaaccat 720
   tacctgtcga cacaatctgc cctttcgaaa gatcccaacg aaaagcgtga ccacatggtc 780
   cttcttgagt ttgtaactgc tgctgggatt acacatggca tggatgagat ctgtagtgtt 840
   gatttgggtt ccggaactga atccagtact gcctcttcta acgcttcggg gtcttcttcc 900
   aagtctaact ccggctcttc tggttcttcc agttcttctt cttcttcttc agcttcatct 960
   tcatcttcta gcaagaagaa tgctgccacc aacgttaaag ctaacttagc acaagtggtc 1020
   tttacctcca tcatttcctt atccattgcc gctggtgtcg gttttgcttt ggtttaaaaa 1080
   gcttcgacac atacataata actcgataag ccgcgg                           1116
<210> 2
   <211> 1236
   <212> DNA
   <213> Saccharomyces cerevisiae
<400> 2
   actagtatgc ttttgcaagc tttccttttc cttttggctg gttttgcagc caaaatatct 60
   gcatcaaaga gctcgcatgc ctgcaggtcg actctagagg atccccgggt accggtagaa 120
   aaaatgagta aaggagaaga acttttcact ggagttgtcc caattcttgt tgaattagat 180
   ggtgatgtta atgggcacaa attttctgtc agtggagagg gtgaaggtga tgcaacatac 240
   ggaaaactta cccttaaatt tatttgcact actggaaaac tacctgttcc atggccaaca 300
   cttgtcacta cgttaactta tggtgttcaa tgcttttccc gttatccgga tcatatgaaa 360
   cggcatgact ttttcaagag tgccatgccc gaaggttatg tacaggaacg cactatatct 420
   ttcaaagatg acgggaacta caagacgcgt gctgaagtca agtttgaagg tgataccctt 480
   gttaatcgta tcgagttaaa aggtattgat tttaaagaag atggaaacat tctcggacac 540
   aaactcgagt acaactataa ctcacacaat gtatacatca cggcagacaa acaaaagaat 600
   ggaatcaaag ctaacttcaa aattcgccac aacattgaag atggatccgt tcaactagca 660
   gaccattatc aacaaaatac tccaattggc gatggccctg tccttttacc agacaaccat 720
   tacctgtcga cacaatctgc cctttcgaaa gatcccaacg aaaagcgtga ccacatggtc 780
   cttcttgagt ttgtaactgc tgctgggatt acacatggca tggatgagat ctctgaatcc 840
   gctgccgcca tttctcaaat cactgacggt caaatccaag ctactaccac tgctaccacc 900
   gaagctacca ccactgctgc cccatcttcc accgttgaaa ctgtttctcc atccagcacc 960
   gaaactatct ctcaacaaac tgaaaatggt gctgctaagg ccgctgtcgg tatgggtgcc 1020
   ggtgctctag ctgctgctgc tatgttgtta taagaaatct ctgatttttt ataatatcta 1080
   tatggctttt ttcaaaattt tcggttttac taggtaagtg tttgattctt ttcttttcgt 1140
   taatatattt ttacacataa ttttaaataa tttttgttat tttgaatagg tagataccat 1200
   aaaaataaaa cactttttac tttaacgagt ccgcgg                          1236